(19)

Europäisches Patentamt

European Patent Office

Office européen des brevets

(11)  **EP 4 516 092 A1**

(12)  **EUROPEAN PATENT APPLICATION**

(43) Date of publication:
**05.03.2025  Bulletin 2025/10**

(21) Application number: **24020267.1**

(22) Date of filing: **13.08.2024**

(51) International Patent Classification (IPC):
*A01H 3/00* (2006.01)        *A01H 3/04* (2006.01)
*A01H 5/06* (2018.01)        *A01H 5/12* (2018.01)
*A01H 6/06* (2018.01)        *A01H 6/20* (2018.01)
*A01N 59/16* (2006.01)      *A01N 63/20* (2020.01)

(52) Cooperative Patent Classification (CPC):
A01H 3/00; A01H 3/04; A01H 5/06; A01H 5/12;
A01H 6/06; A01H 6/20; A01H 6/206

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **15.08.2023  PL 44583523**

(71) Applicant: **Uniwersytet Mikolaja Kopernika w
Toruniu
87-100 Torun (PL)**

(72) Inventors:
  • **Dabrowska, Grazyna Barbara
    87-100 Torun (PL)**

  • **Mierek-Adamska, Agnieszka
    87-100 Torun (PL)**
  • **Kulasek, Milena
    87-100 Torun (PL)**
  • **Konieczna, Wiktoria
    87-100 Torun (PL)**
  • **Turkan, Sena
    87-100 Torun (PL)**
  • **Antoszewski, Marcel
    54-107 Wroclaw (PL)**
  • **Wlodarczyk, Katarzyna
    47-133 Jemielnica (PL)**
  • **Szydlowska-Czerniak, Aleksandra
    87-100 Torun (PL)**

(54)  **THE METHOD OF OBTAINING PLANT SEEDLINGS OF AN INCREASED ZINC CONTENT**

(57)    The subject of the application is the method of obtaining seedlings of plants of an increased zinc content enhancing resistance to viruses, and applied for the purposes of consumption.

EP 4 516 092 A1

**Description**

The subject of the invention is a method of obtaining plant seedlings of an increased zinc content enhancing resistance to viruses, and applied for the purposes of consumption.

By the year 2050, the world population will have reached the amount of 9 billion people and, as expected, most of the growth will occur in the developing countries. According to the Food and Agriculture Organization of the United Nations, in 2020, about 9.9% of the total human population was affected by lack of food security. The occurrence of hunger significantly increased in frequency within only one year due to the COVID-19 pandemic. Another 17.2% of people experience moderate lack of food security (The State of Food Security and Nutrition in the World 2021; FAO, Ed.; FAO, IFAD, UNICEF, WFP and WHO: Rome, 2021; ISBN 978-92-5-134325-8), which means they need to choose between the quality and the quantity of food, owing to which they experience "hidden hunger", that is micronutrient deficiency, resulting from inadequate diet in which intake or absorption of the key vitamins and minerals is too low to provide good health condition. Together with vitamin A, iron, and iodine, zinc is one of the micronutrients most frequently mentioned in the context of "hidden hunger". Zinc is necessary for the all plants and animals as it is a cofactor for thousands of proteins including enzymes of all the six classes. Zinc finger proteins which have a broad range of molecular and cellular functions including transcription regulation, DNA repair, ubiquitin-dependent proteomic degradation, signal transduction, cell proliferation, differentiation, migration, and apoptosis are most abundant of them (Maret W. 2013, Advances Nutrition 4, 82-91). Moreover, zinc is an essential participant in antioxidative mechanisms due to among others the NADPH-oxidase enzyme inhibition, glutamate-cysteine ligase expression induction; it is also the Cu, Zn superoxide dismutase (ZnCuSOD) cofactor (Marreiro D. i in. 2017, Antioxidants 6, 24). Therefore, zinc deficiency results in serious ailments such as growth and developmental retardation, anorexia, and weakening of the immune system (Prasad A.S. 2009, J. Am. Coll. Nutr. 28, 257-265), because zinc is of the fundamental importance in the production, differentiation, and functioning of immune cells, e.g. neutrophils, natural killer cells, macrophages, T and B lymphocytes, as well as in the production of cytokines and in phagocytosis (Gammoh N.Z. 2019, Nutrition and Immunity 127-158; Prasad A.S. 2008, Mol. Med. 14, 353-357). According to estimations, the deficiency of this single element leads to 0.4 million deaths among children under 5 years of age annually (Black R.E. i in. 2008. Lancet 371, 243-260). Antiviral activity of zinc against numerous types of viruses, both by direct antiviral activity and by immune system stimulation was demonstrated (Read S.A. i in. 2019, Adv. Nutr. 10, 696-710; Wessels I. 2020, Front. Immunol. 11, 1712). Hence, in view of the SARS-CoV-2 pandemic, a struggle with zinc deficiency acquires even more significance. Meat (and seafood) is the main source of zinc in the diet of people living in developed countries. However, in areas where the risk of zinc deficiency is highest, diet is primarily based on plant consumption (Kumssa D.B. i in. 2015. Sci. Rep. 5, 1-11; Lim K.H.C. i in. 2013, Nutrients 5, 3184). The main problem of the plant-based diets is related to a highly limited content and/or bioavailability of zinc from grains such as rice, wheat, corn, barley, sorgo, rye, and oats. Tackling the issue of zinc deficiency by the diversification of food consumption, i.e. higher meat and/or seafood consumption, is currently impossible for the majority of the most affected part of population in countries with low or medium income. Moreover, to counteract the adverse effect of the livestock industry on climate changes, the developed countries are expected to reduce meat consumption and follow more plant-based diet. Thus, developing an effective method for the enrichment of edible parts of plants with zinc is so essential. Biofortification, the concept of the basic crop plants enrichment with necessary nutrients aimed at increasing the nutritional value of food is one of the most promising solutions (Clemens S. 2014, Plant Sci. 225, 52-57).

In order to develop new croppers either by genetic modifications or by traditional cultivation it is essential to thoroughly understand molecular mechanisms underlying the uptake, transport, and storage of metals in plants.

Defense reactions in plants are stimulated by contact with both abiotic as well as biotic factors. A non-specific response of plants to stress is possible owing to the existence of the cell wall and production of secondary metabolites. Plants are also equipped with mechanisms of the so-called priming memory which due to the experienced stress enables a faster and more intense plant reaction to other undesirable factors (Balmer A. i in. 2015, Trends Plant Sci 20, 7; Hossain M.A. i in. 2015, Front Plant Science 6, 420). The reaction and tolerance to stress is diversified and dependent on a plant species. To induce stress memory, plants are subject to priming, i.e. the induction of the physiological condition which allows the plant to implement a faster and stronger defense reaction to stress in comparison to plants which did not undergo that procedure (Balmer A. i in. 2015, Trends Plant Sci 20, 7). That natural ability of plants to memorize stress is applied to stimulate seed germination. Seed priming which often increases tolerance of germinating seeds to stress factors (e.g. cold, salinity, drought, high temperature) is one of such procedures. The process is commonly applied in agriculture, horticulture, and forestry. Priming seeds of plants representing different species increased the percentage of germinated seeds by 11% in average and shortened the average germination time by 36% in comparison to non-primed seeds. According to literature, the efficiency of primed seeds depends on a plant species, seed quality, seed priming procedure, and germination conditions. Priming improves the vigor and viability of plants, the power and synchronization of germination, and enhances the vigor of seedlings. During priming under controlled conditions, seed hydration occurs to a degree insufficient for a radicle to break through the seed coating (Bailly C. i in. 2008, Comptes Rendus Biologies 331, 10, 806 - 814; Kubala S. i in. 2013, Post. Biol. Kom. 40, 2, 215-230). Seed priming induces a number of signal pathways, which is dependent on the priming conditions. Positive priming effects can result from the induction of biochemical cell repair mechanisms which restore their integrity through reactivating their metabolic functioning, synthesizing nucleic acids and proteins as well as improving the antioxidative system (di Girolamo G., Barbanti L. 2012, Italian J Agron 7; Balmer A. i in. 2015, Trends Plant Sci 20, 7).

The most frequently applied methods involve hormonal, hydro-, osmo-, and biopriming, which are based on the incubation of seeds in a growth regulator solution, water, osmotically active solution, or microorganisms solution, respectively (Ibrahim E.A. 2015, J Plant. Physiol. 192, 38-46; Sivritepe H.O., Sivritepe N. 2016, Not. Bot. Hort. Agrobot. 44, 2, 399-403). Storing seeds in the above mentioned solutions results in their swelling. The duration of priming must be carefully controlled since the radicle must not break through the seed coating (Jisha K.C. i in. 2012, Acta Physiol. Plantarum 35, 1981-1396; Kubala S. i in. 2013, Post. Biol. Kom. 40, 2, 215-230). Osmotically active substances, i.e. polyethylene glycol (PEG), mannitol, glycerin, and NaCl are applied for seed osmopriming (Ventura L. 2012, Plant Physiol. Biochem. 60, 196-206). Hydropriming and osmopriming can be successfully employed as mechanisms of providing chemical substances such as growth regulators, stimulants, or bioactive compounds. Such an approach involves the use of a broad range of compounds, both natural and synthetic, e.g. antioxidants (ascorbic acid, proline), hydrogen peroxide, or fungicidal agents (Wojtyla L. i in. 2016, J Pant Physiol. 203, 116-126). It has been demonstrated that priming with the use of $H_2O_2$ improves plant tolerance to adverse conditions by stimulating the expression of protein coding genes which participate in RTF detoxification and stress response pathways activation. The result of priming characterized by an increase in antioxidant properties of plants can correlate not only with a high level of enzymatic antioxidants transcription but also with glutathione production (Hossein M.A. i in. 2015, Front Plant Sci. 6, 420; Savvides A. i in. 2015, Trends Plant Sci. 21, 4, 329-340). Mahdavi B. and Rahimi A. (2013, EurAsia J BioSci 7, 69-76) demonstrated that the treatment of Indian caraway (*Carum copticum*) seeds with chitosan, a chitin derivative, significantly contributed to the improvement of the studied parameters. Priming with the use of 0.2% chitosan solution caused that germination occurred in 80% of seeds while for the seeds from the control sample it was only 52%, and the germination speed as well as seed vigor index increased together with an increase in the concentration of chitosan.

Priming with the use of microorganisms is also one of the priming methods. In the following solution, priming with the use of the *Lactobacillus* bacteria which naturally inhabit the human intestine was applied. These bacteria are characterized by their resistance to the activity of gastric and bile acids and the ability of the strain cells to bind to the surface of the mucous membrane in the intestine due to the presence of the intestinal villi which increase the adhesive capability. The *Lactobacillus* cells produce specific proteins that influence the intestinal epithelium, which results in lowering its permeability. This influences the formation of an antibacterial barrier and brings about the mucous antiinflammatory effect by the direct action on macrophages, dendritic cells, and CD4+lymphocytes, which results in a decrease in the production of TNF inflammatory cytokines, IL-2, IL-4 (Pagnini C. 2023. Microorganisms 11, 6, 1381; Gorbach S. 1996. Nutrition Today 31, suppl. 1, 2-4).

The subject of the invention is the method of sowing material preparation by treating seeds (priming) with zinc sulfate and/or bacteria of the *Lactobacillus* strain and hydroponic cultivation of plants (cress, dill, radish) for consumption which as a result of the treatment will be characterized by an increased zinc content. It will allow the production of food of a high nutritional value, which will influence the intake of zinc and fight with "hidden hunger" of humans, and thus it has an impact on an improvement in the ability of the human body to resist diseases, including infections caused by viruses.

The aim of the invention is to develop the method of preparing sowing material that will allow obtaining the material of an increased zinc content.

The subject of the invention is the method of obtaining plant seedlings of an increased zinc content which is based on the sterilization of vegetable seeds or herbs preferably dill Anethum graveolens L., cress Lepidium sativum L. using usual methods and priming in a glass flask filled with ZnSO4 in the amount from 0.05 to 3 mM preferably 0.125 mM per each 1 g of seeds, preferably in the time from 1 h to 10 h most preferably 5 h, simultaneously stirred, and next seeds transferring to a Petri dish covered with dry filter paper and leaving preferably for the time from 2 to 24 h most preferably 12 h at the temperature from 25 to 45 °C preferably 37 °C, and next drying the Petri dishes with seeds in an incubator preferably for the time from 24 to 120 h most preferably for 72 h at the temperature form 15 to 35 °C preferably 22 °C, and storing the thus prepared seeds using usual methods.

The subject of the invention is the method of obtaining plant seedlings of an increased zinc content which is based on that the vegetable or herb seeds preferably radish Raphanus sativus L. are sterilized using usual methods and undergo priming in a glass flask filled with ZnSO4 in the amount from 0.05 to 3 mM preferably 0.25 mM per each 1 g of seeds, preferably in the time from 1 to 10 h most preferably 5 h, simultaneously stirred, and next seeds are transferred to a Petri dish covered with dry filter paper and left preferably for the time from 2 to 24 h most preferably 12 h at the temperature from 25 to 45 °C preferably 37 °C, and next the Petri dishes with seeds are dried in an incubator preferably in the time from 24 h to 120 h most preferably for 72 h at the temperature from 15 to 35 °C preferably 22 °C, and the thus prepared seeds are stored using usual methods.

The subject of the invention is the method of obtaining plant seedlings of an increased zinc content which is based on that the vegetable or herb seeds preferably radish Anethum graveolens L., cress Lepidium sativum L, are sterilized using usual methods and undergo priming in a glass flask in a mixture of ZnSO4 in the amount from 0.05 to 3 mM preferably 0.125 mM and Lactobacillus of the optical density (OD) in the range from 0.2 to 1.0 preferably OD=0.5 in the amount from 10 to 40 ml preferably 15 ml per each 1g of seeds preferably in the time from 1.0 to 10 h most preferably 5 h, simultaneously stirred, and next seeds are transferred to a Petri dish covered with dry filter paper and left preferably for the time from 2 to 24 h most preferably 12 h at the temperature from 25 to 45 °C preferably 37 °C, and next the Petri dishes with seeds are dried in an incubator preferably in the time from 24 h to 120 h most preferably for 72 h at the temperature from 15 to 35°C preferably 22 °C, and the thus prepared seeds are stored using usual methods.

The subject of the invention is the method of obtaining plant seedlings of an increased zinc content which is based on that the vegetable seeds preferably radish Raphanus sativus L. are sterilized using usual methods and undergo priming in a glass flask in a mixture of ZnSO4 in the amount from 0.05 to 3 mM preferably 0.25 mM and Lactobacillus of the optical density (OD) in the range from 0.2 to 1.0 preferably OD=0.5 in the amount from 10 to 40 ml preferably 20 ml per each 1g of seeds preferably in the time from 1.0 to 10 h most preferably 5 h, simultaneously stirred, and next seeds are transferred to a Petri dish covered with dry filter paper and left for the time from 2 to 24 h most preferably for 12 h at the temperature from 25 to 45°C preferably 37°C, and next the Petri dishes with seeds are dried in an incubator preferably in the time from 24 h to 120 h most preferably for 72 h at the temperature from 15 to 35 °C preferably 22 °C, and the thus prepared seeds are stored using usual methods.

Example I.

Materials and methods

For the purposes of research, seeds of three vegetable and herb species were chosen through tests (i.e. dill (*Anethum graveolens* L., Vilmorin Garden Company, LTD), cress (*Lepidium sativum* L., Centrum Ogrodnicze Bronisze Company) and radish (*Raphanus sativus* L., W. Legutko Company, the Saxa 2 species). The method of sterile seeds treatment with chemical compounds ($ZnSO_4$) and biological means (microorganisms of the *Lactobacillus* species responsible for the production of vitamin B6) was developed. The results of the research indicated that zinc in the form of $ZnSO_4$ is effectively absorbed by plants and positively influences the germination and growth of the tested plants. For the purposes of seed priming, 0.25 mM of $ZnSO_4$ was used, and in the cases of dill and cress, 0.125 mM of the above mentioned compound solution was used. Additionally, the seeds underwent priming with a bacterial suspension of *Lactobacillus* of the optical density of 0.5. The third experimental option involved seeds treated with zinc and *Lactobacillus* bacteria solutions; seeds treated with the 0.9% NaCl solution, distilled water as well as seeds without treatment constituted the control group. Finally, biological and chemical factors influencing the improvement of zinc content in plants were selected.

The *Lactobacillus* strain cultivation was performed on a liquid MRS medium (1% pepton, 1.2% yeast extract, 2% D(+)-glucose, 0.2% $K_2HPO_4$, 0.5% sodium acetate, 0.2% diammonium citrate, 0.02% $MgSO_4 \cdot 7H_2O$, 0.005% MnSO4·4-$H_2O$). In order to prepare the bacteria for application in the priming process, they were cultivated in a flask filled with 100 ml of the sterile MRS culture medium, at the temperature of 37°C with stirring for 48 h. Next, the culture was centrifuged (8000 x g, 15 min) and the supernatant was removed. The cells were rinsed with 0.9% NaCl and centrifuged again. The supernatant was discarded, and the cells were suspended again in 0.9% NaCl and diluted to $OD_{600}$=0.5.

In order to prepare the plant material, 2 g of radish, dill, and cress seeds were surface sterilized by stirring in a mixture of 96% EtOH and 30% $H_2O_2$ (v:v, 1: 1) for 5 minutes, and next the seeds were rinsed fivefold with sterile distilled water. Subsequently, the seeds were evenly distributed to five flasks containing 1) sterile water, 2) 0.9% NaCl, 3) sterile water with 0.25 mM $ZnSO_4$ (in the case of radish seeds) or 0.125 mM $ZnSO_4$ (in the case of dill and cress seeds), 4) *Lactobacillus*, $OD_{600}$=0.5 in 0.9% NaCl, 5) *Lactobacillus* 0.25 mM $ZnSO_4$ (in the case of radish) or 0.125 mM $ZnSO_4$ (in the case of dill and cress seeds). Next, the seeds remained in the solutions for 5 h and were stirred at room temperature. After that, the seeds were transferred to sterile Petri dishes covered with dry filter paper and left overnight at the temperature of 37°C. Next day, the Petri dishes filled with the seeds were placed in an incubator to further dry the seeds for another three days at the temperature of 22 °C. The thus prepared seeds are stored using usual methods.

The completely dry seeds were subsequently applied as the material for further experiments in which the treated seeds (n=15) were seeded on Petri dishes wetted with 3.5 ml sterile distilled water. Non-primed seeds constituted the control sample. The radish and cress seeds germinated in the dark for one day and were then transferred to a light chamber and left for another 6 days and cultivated at 25 °C, in the photoperiod of 16h in light and 8h in the dark, at the photosynthetically active radiation (PAR)=100 mmol photons $m^{-2}$ $s^{-1}$. The dill seeds germinated in the dark for 3 days and were then transferred to a light chamber and left for another 11 days and cultivated at 25 °C, in the photoperiod of 16h in light and 8h in the dark, at PAR=100 mmol photons $m^{-2}$ $s^{-1}$. The numbers of the germinating seeds were calculated daily. Next, the lengths of roots and shoots were measured as well as their fresh and dry biomass. The experiment was performed three times.

The description of the chlorophyll content determination

The amount of the photosynthetic pigments was measured by crushing 10 mg of dry plant tissue with the use of TissueLyser LT. 100 mg of $CaCO_2$ and 1 ml of 96% EtOH were added to the crushed material. The samples were stirred for 1 min by shaking and then centrifuged (10 000 x g, 5 min, 4°C). The supernatant was transferred to a flask of the Falcon type. The remaining sediment was rinsed with 1 ml of 96% EtOH and centrifuged, and the supernatant was collected again. The process was repeated until the supernatant was colorless. 200 µl of the collected supernatant was used to measure absorbance (A) at $\lambda$=470, 648, and 664 nm. The amounts of the a and b chlorophyll as well as carotenoids were calculated according to the Lichtenthaler and Wellburn (1983)

$$Chl.\ a = 13.36\ A_{664} - 5.19\ A_{648}$$

$$Chl.\ b = 27.43\ A_{648} - 8.12\ A_{664}$$

$$Car = (1000\ A_{470} - 2.13\ Chl.\ a - 97.64\ Chl.\ b)/209$$

where $A\lambda$ is the value of absorbance for the wavelength $[\lambda]$, Chl. a is the amount of chlorophyll a, Chl. b is the amount of chlorophyll b, and Car is the total carotenoids concentration.

Results

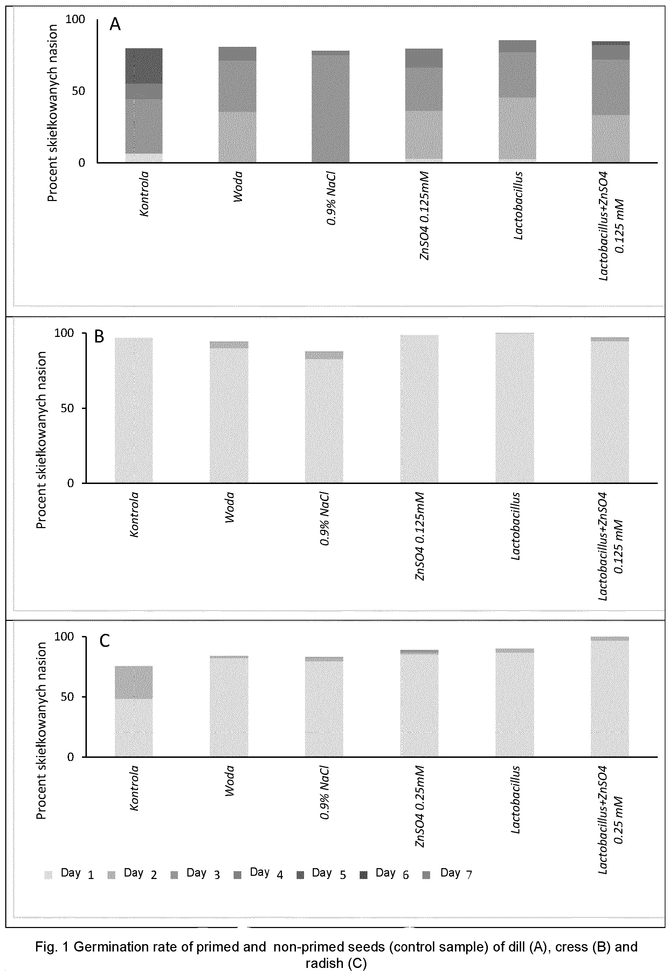

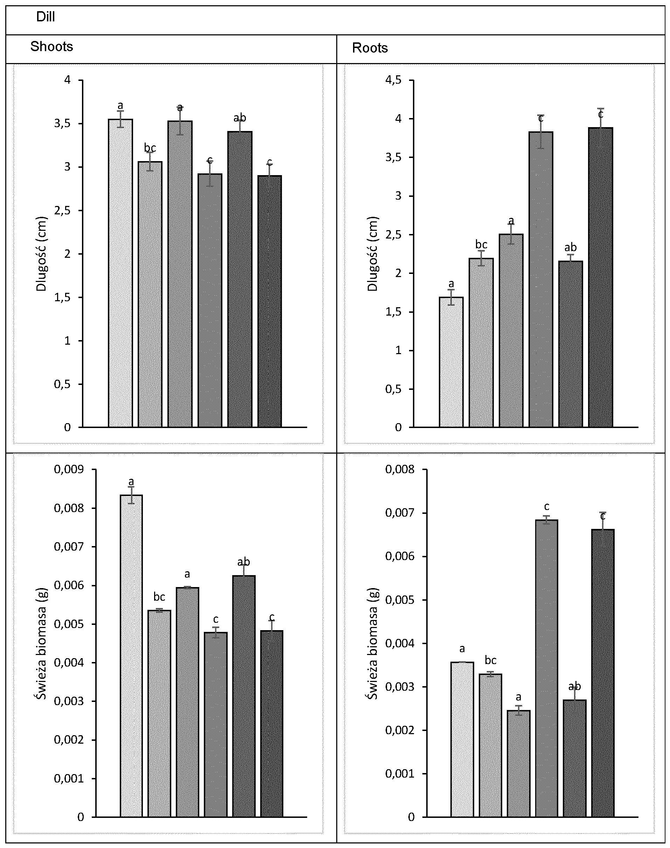

The impact of the seed treatment on the biometric and physiological parameters of the plants was estimated. The cress seeds which underwent priming (all the analysed factors) germinated to a degree higher than the control seeds without priming. (Fig. 1). In the case of the radish, the highest amount of seeds germinated after priming in 0.25 mM $ZnSO_4$ and $ZnSO_4$ + *Lactobacillus* (Fig. 1C). In the case of the dill, the highest percentage of the germinated seeds was attained for the seeds after priming with 0.125 mM $ZnSO_4$ + *Lactobacillus* (Fig. 1A). In the case of the cress, the highest percentage of the germinated seeds was attained for the seeds after treatment with bacteria of the *Lactobacillus* strain (Fig. 1B).

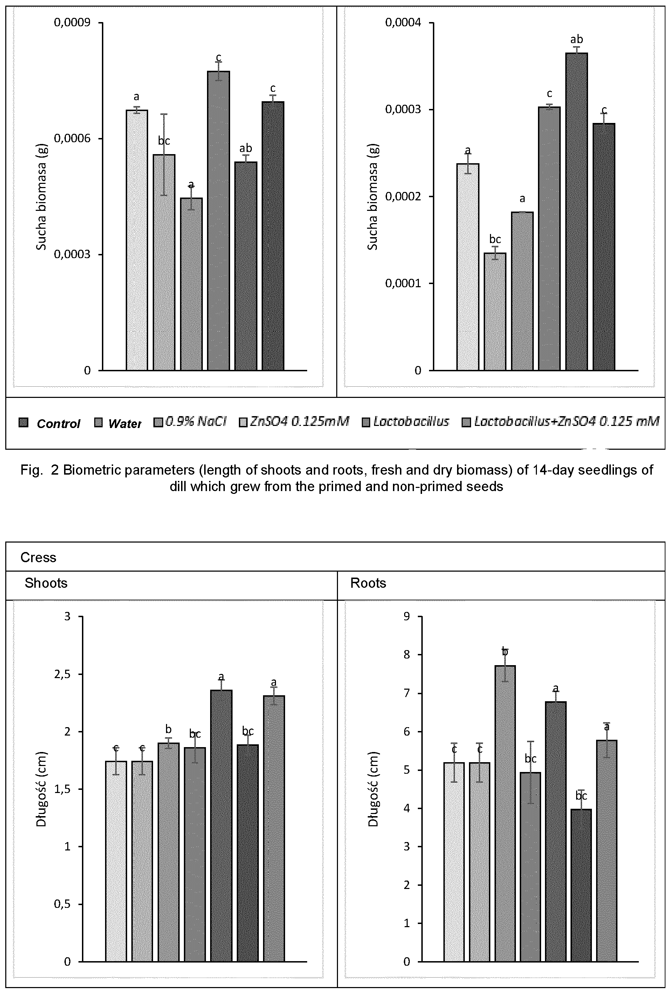

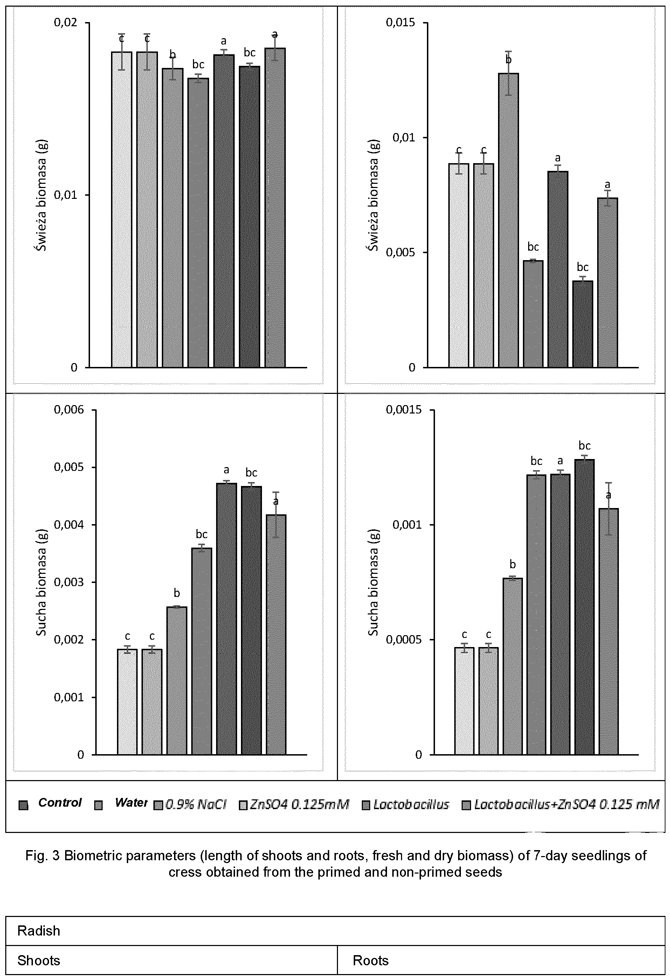

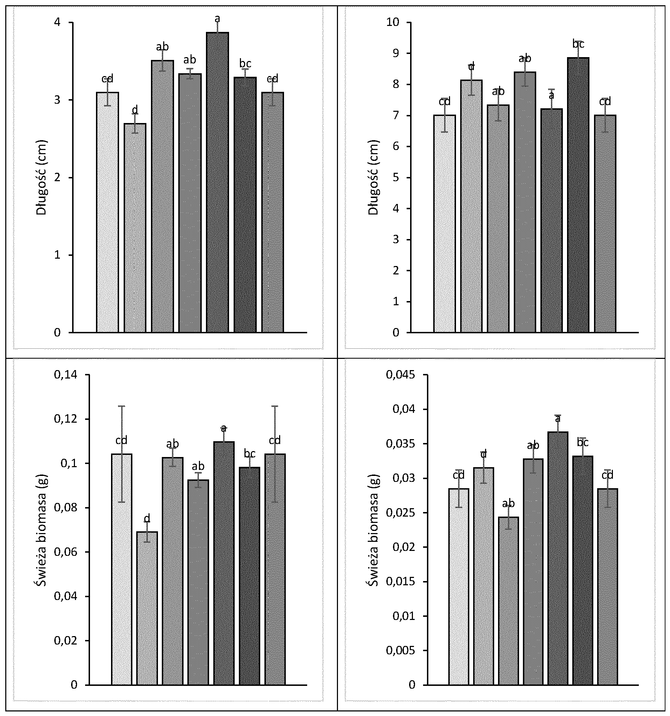

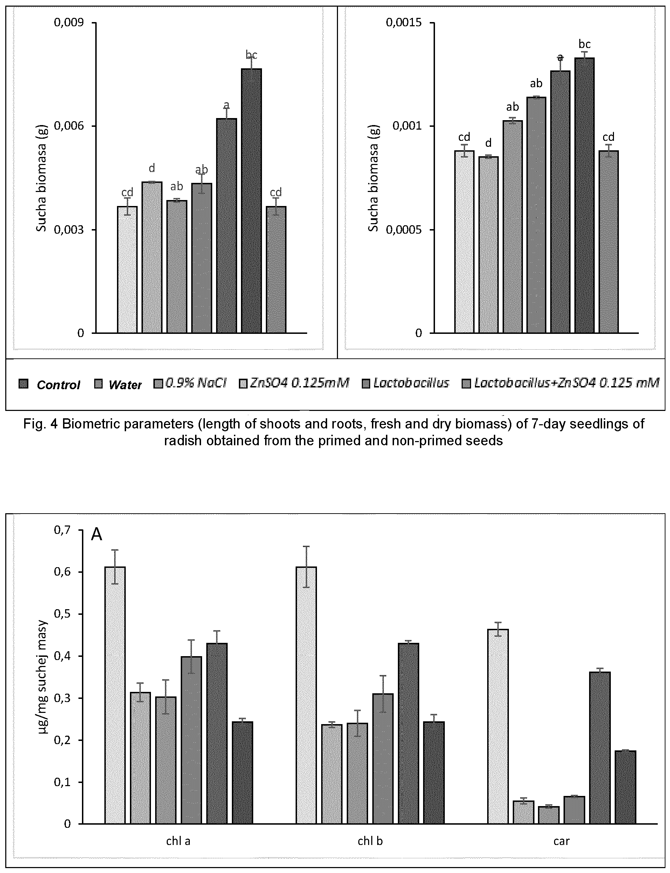

In the case of the dill, the presence of $ZnSO_4$ and $ZnSO_4$ + *Lactobacillus* resulted in the appearance of the seedling roots of twice the length and the fresh biomass of the roots was threefold higher (Fig. 2). In the case of the cress, $ZnSO_4$ ions and $ZnSO_4$ + *Lactobacillus* applied for priming stimulated the elongation of the shoots and dry biomass of organs (Fig. 3). Also, a twofold increase in the dry biomass of the cress roots was observed in the following variants: $ZnSO_4$, *Lactobacillus,* and $ZnSO_4$ + *Lactobacillus* (Fig. 3). A positive influence of $ZnSO_4$ ions and $ZnSO_4$ + *Lactobacillus* was demonstrated, which resulted in an increase in the length of the shoots and roots as well as dry and fresh biomass of these organs in the radish (Fig. 4). The analysis of the biometric parameters indicated that zinc combined with the bacteria of the *Lactobacillus* strain are promising factors which can be applied for seed priming and constitute one of the innovative methods of preparing sowing material of plants used for consumption as several-day seedlings.

The content of the photosynthetic pigments in the plant material (cress, dill, and radish seedlings) obtained from primed and non-primed seeds was measured.

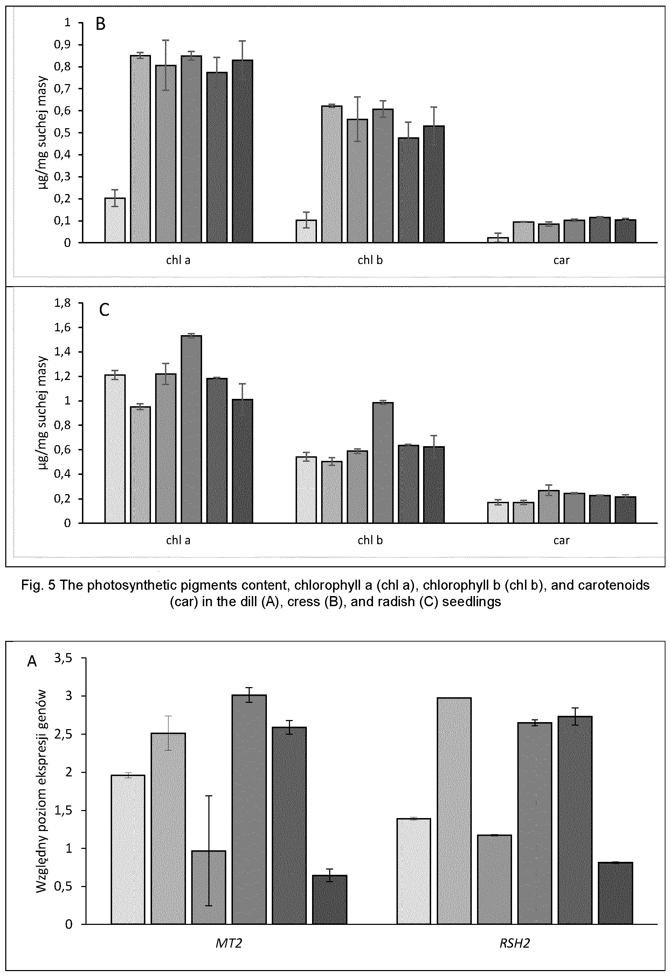

According to observations, the photosynthetic pigments content (chlorophyll a/b and carotenoids) reached the highest value in the dill grown from the non-primed seeds (Fig. 5A). In the cress, the photosynthetic pigments content was higher in plants grown from the primed seeds (Fig. 5B). No changes in the chlorophyll content were observed for the radish seedlings (Fig. 5C), which indicates that the examined factors did not cause stress in the plants.

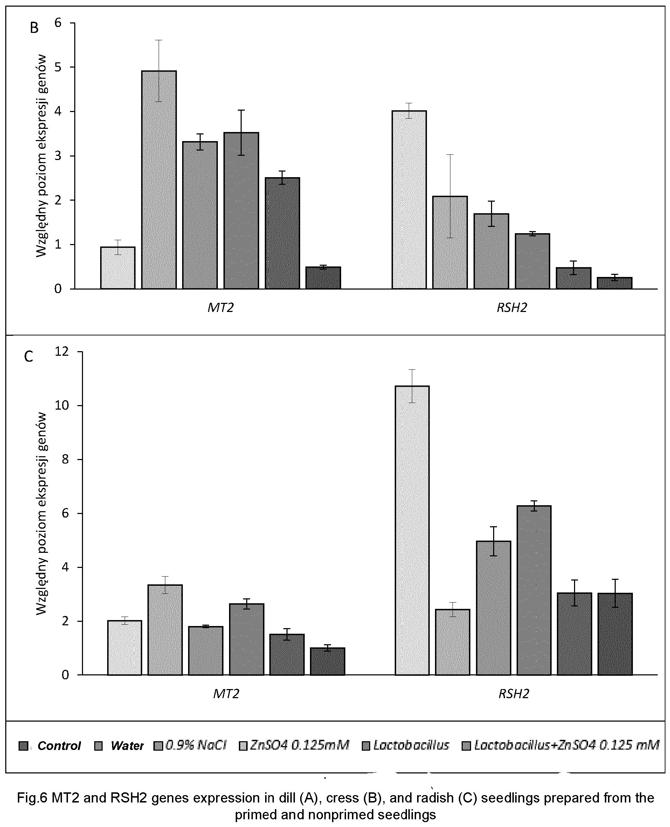

The experiments were performed in which the *MT* i *RSH* genes expression was analyzed, which allowed the selection of the molecular zinc content marker in plants. It was demonstrated that the melatonin type 2 (*MT2*)in all the analyzed plant species (radish, crest, and dill) changed in the presence of zinc in seeds and seedlings. The lowest level of the examined genes expression was observed for the samples treated with the mixture of bacteria and zinc, which indicates that the presence of the two factors reduces the stress level in these plants (Fig. 6).

The conducted examinations showed that priming seeds with the above mentioned factors results in not only the acceleration of the process of plant germination and growth, which is particularly essential from the food producers' point of view. The solution described above also affects the zinc content in plants, which guarantees that the food produced is of a higher nutritional value, which in turn is important for the consumer. Moreover, an efficient methodology of growing three plants consumed in the form of sprouts or seedlings was developed and tested.

Example II.

The method of obtaining plant seedlings of an increased zinc content is that the seeds of dill, *Anethum graveolens* L., in the amount of 30g are sterilized using usual methods and primed in a glass flask in $ZnSO_4$ in the amount of 0.05 mM per each 1 g of the seeds for 1h simultaneously stirring, and next the seeds are transferred on a Petri dish covered with dry filter paper and left for 2 h at the temperature of 25°C, and next the Petri dishes with the seeds are dried in an incubator for 24 h at the temperature of 15 °C, and the thus prepared seeds are stored using usual methods.

The obtained seeds were characterized by the same properties as those in Example I. Example III.

The method of obtaining plant seedlings of an increased zinc content is that the seeds of cress, *Lepidium sativum* L., in the amount of 15g are sterilized using usual methods and primed in a glass flask in $ZnSO_4$ in the amount of 3mM per each 1 g of the seeds for 10 h simultaneously stirring, and next the seeds are transferred on a Petri dish covered with dry filter paper and left for 24 h at the temperature of 45°C, and next the Petri dishes with the seeds are dried in an incubator for 120 h at the temperature of 35 °C, and the thus prepared seeds are stored using usual methods.

The obtained seeds were characterized by the same properties as those in Example I.

Example IV

The method of obtaining plant seedlings of an increased zinc content is that the seeds of radish, *Raphanus sativus* L., are sterilized using usual methods and primed in a glass flask in $ZnSO_4$ in the amount of 0.05 mM per each 1 g of the seeds for 1h simultaneously stirring, and next the seeds are transferred on a Petri dish covered with dry filter paper and left for 2 h at the temperature of 25°C, and next the Petri dishes with the seeds are dried in an incubator for 24 h at the temperature of 15 °C, and the thus prepared seeds are stored using usual methods.

The obtained seeds were characterized by the same properties as those in Example I.

Example V

The method of obtaining plant seedlings of an increased zinc content is characterized in that the radish, *Raphanus sativus* L., in the amount of 60g are sterilized using usual methods and primed in a glass flask in $ZnSO_4$ in the amount of 3 mM per each 1 g of the seeds for 10 h simultaneously stirring, and next the seeds are transferred on a Petri dish covered with dry filter paper and left for 24 h at the temperature of 45°C, and next the Petri dishes with the seeds are dried in an incubator for 120 h at the temperature of 35 °C, and the thus prepared seeds are stored using usual methods.

The obtained seeds were characterized by the same properties as those in Example I.

Example VI. The method of obtaining plant seedlings of an increased zinc content is that the seeds of dill, *Anethum graveolens* L., in the amount of 80g are sterilized using usual methods and primed in a glass flask in the mixture of $ZnSO_4$ in the amount of 0.05 mM and *Lactobacillus* of the optical density OD=0.2 in the amount of 10 ml per each 1 g of the seeds for 1 h simultaneously stirring, and next the seeds are transferred on a Petri dish covered with dry filter paper and left for 2 h at the temperature of 25 °C, and next the Petri dishes with the seeds are dried in an incubator for 24 h at the temperature of 15 °C, and the thus prepared seeds are stored using usual methods.

The obtained seeds were characterized by the same properties as those in Example I.

Example VII. The method of obtaining plant seedlings of an increased zinc content is that the seeds of cress, *Lepidium sativum* L., in the amount of 25 g are sterilized using usual methods and primed in a glass flask in a mixture of $ZnSO_4$ in the amount of 3 mM and *Lactobacillus* of the optical density OD=1.0 in the amount of 40 ml per each 1 g of the seeds for 10 h simultaneously stirring, and next the seeds are transferred on a Petri dish covered with dry filter paper and left for 24 h at the temperature of 45 °C, and next the Petri dishes with the seeds are dried in an incubator for 80 h at the temperature of 35 °C, and the thus prepared seeds are stored using usual methods.

The obtained seeds were characterized by the same properties as those in Example I.

Example VIII. The method of obtaining plant seedlings of an increased zinc content is that the seeds of radish, *Raphanus sativus* L., are sterilized using usual methods and primed in a glass flask in a mixture of $ZnSO_4$ in the amount of 0.05 mM and *Lactobacillus* of the optical density OD=0.2 in the amount of 10 ml per each 1 g of the seeds for 1 h simultaneously stirring, and next the seeds are transferred on a Petri dish covered with dry filter paper and left for 2 h at the temperature of 25°C, and next the Petri dishes with the seeds are dried in an incubator for 24 h at the temperature of 15 °C, and the thus prepared seeds are stored using usual methods.

The obtained seeds were characterized by the same properties as those in Example I.

Example IX. The method of obtaining plant seedlings of an increased zinc content is that the seeds of radish, *Raphanus sativus* L., are sterilized using usual methods and primed in a glass flask in a mixture of $ZnSO_4$ in the amount of 3 mM and *Lactobacillus* of the optical density OD=1.0 in the amount of 40 ml per each 1 g of the seeds for 10 h simultaneously stirring, and next the seeds are transferred on a Petri dish covered with dry filter paper and left for 24 h at the temperature of 45°C, and next the Petri dishes with the seeds are dried in an incubator for 120 h at the temperature of 35 °C, and the thus prepared seeds are stored using usual methods.

The obtained seeds were characterized by the same properties as those in Example I.

**Claims**

1. The method of obtaining plant seedlings of an increased zinc content **characterized in that** seeds of vegetables or herbs preferably dill *Anethum graveolens* L., cress *Lepidium sativum* L. are sterilized using usual methods and primed in a glass flask in $ZnSO_4$ in the amount from 0.05 to 3 mM preferably 0.125 mM per each 1 g of the seeds preferably for 1 h to 10 h most preferably 5 h simultaneously stirring, and next the seeds are transferred on a Petri dish covered with dry filter paper and left preferably for 2 h to 24 h most preferably 12 h at the temperature from 25°C to 45°C preferably 37 °C and next the Petri dishes with the seeds are dried in an incubator preferably for 24 h to 120 h most preferably for 72 h at the temperature from 15°C to 35°C preferably 22 °C and the thus prepared seeds are stored using usual methods.

2. The method of obtaining plant seedlings of an increased zinc content **characterized in that** seeds of vegetables or herbs preferably radish, *Raphanus sativus* L. are sterilized using usual methods and primed in a glass flask in $ZnSO_4$ in the amount from 0.05 to 3 mM preferably 0.25 mM per each 1 g of the seeds preferably for 1 h to 10 h most preferably 5 h simultaneously stirring, and next the seeds are transferred on a Petri dish covered with dry filter paper and left preferably for 2 h to 24 h most preferably 12 h at the temperature from 25°C to 45°C preferably 37°C and next the Petri dishes with the seeds are dried in an incubator preferably for 24 h to 120 h most preferably 72 h at the temperature from 15 °C to 35°C preferably 22°C and the thus prepared seeds are stored using usual methods.

3. The method of obtaining plant seedlings of an increased zinc content **characterized in that** seeds of vegetables or herbs preferably dill *Anethum graveolens* L., cress *Lepidium sativum* L. are sterilized using usual methods and primed in a glass flask in a mixture of $ZnSO_4$ in the amount from 0.05 to 3mM preferably 0.125 mM and *Lactobacillus* of the optical density (OD) in the range from 0.2 to 1.0 preferably OD=0.5 in the amount from 10 ml to 40 ml preferably 15 ml per each 1 g of the seeds preferably for 1 h to 10 h most preferably 5 h simultaneously stirring, and next the seeds are transferred on a Petri dish covered with dry filter paper and left preferably for 2 h to 24 h most preferably 12 h at the temperature from 25°C to 45°C preferably 37°C, and next the Petri dishes with the seeds are dried in an incubator preferably from 24 h to 120 h most preferably 72 h at the temperature from 15°C to 35°C preferably 22°C, and the thus prepared seeds are stored using usual methods.

4. The method of obtaining plant seedlings of an increased zinc content **characterized in that** seeds of vegetables preferably radish *Raphanus sativus* L. are sterilized using usual methods and primed in a glass flask in a mixture of $ZnSO_4$ in the amount from 0.05 to 3mM preferably 0.25 mM and *Lactobacillus* of the optical density (OD) in the range from 0.2 to 1.0 preferably OD=0.5 in the amount from 10 ml to 40 ml preferably 20 ml per each 1 g of the seeds preferably for 1 h to 10 h most preferably 5 h simultaneously stirring, and next the seeds are transferred on a Petri dish covered with dry filter paper and left preferably for the time from 2 h to 24 h most preferably 12 h at the temperature from 25°C to 45°C preferably 37°C, and next the Petri dishes with the seeds are dried in an incubator preferably in the time from 24 h to 120 h most preferably for 72 h at the temperature from 15°C to 35°C preferably 22°C, and the thus prepared seeds are stored using usual methods.

Fig. 1 Germination rate of primed and non-primed seeds (control sample) of dill (A), cress (B) and radish (C)

Dill

Shoots — Roots

Fig. 2 Biometric parameters (length of shoots and roots, fresh and dry biomass) of 14-day seedlings of dill which grew from the primed and non-primed seeds

Fig. 3 Biometric parameters (length of shoots and roots, fresh and dry biomass) of 7-day seedlings of cress obtained from the primed and non-primed seeds

| Radish | |
|---|---|
| Shoots | Roots |

Fig. 4 Biometric parameters (length of shoots and roots, fresh and dry biomass) of 7-day seedlings of radish obtained from the primed and non-primed seeds

Fig. 5 The photosynthetic pigments content, chlorophyll a (chl a), chlorophyll b (chl b), and carotenoids (car) in the dill (A), cress (B), and radish (C) seedlings

Fig.6 MT2 and RSH2 genes expression in dill (A), cress (B), and radish (C) seedlings prepared from the primed and nonprimed seedlings

**EP 4 516 092 A1**

Europäisches Patentamt

European Patent Office

Office européen des brevets

# EUROPEAN SEARCH REPORT

**Application Number**

EP 24 02 0267

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MIRSHEKARI BAHRAM ET AL: "POSITIVE RESPONSE OF ESSENTIAL OIL PERCENTAGE AND ITS YIELD TO SEED PRE-TREATMENT WITH SOME OF MAJOR MICRO-ELEMENTS CASE STUDY: ANETHUM GRAVEOLENS AS A MEDICINAL PLANT", INDIAN JOURNAL OF FUNDAMENTAL AND APPLIED LIFE SCIENCES, vol. 4, no. 2, 1 April 2014 (2014-04-01), pages 453-461, XP093200771, ISSN: 2231-6345 Retrieved from the Internet: URL:https://www.cibtech.org/J-LIFE-SCIENCES/PUBLICATIONS/2014/Vol-4-No-2/JLS-070-086-MIRSHEKARI-POSITIVE-ELEMENTS.pdf> * the whole document * | 1-4 | INV. A01H3/00 A01H3/04 A01H5/06 A01H5/12 A01H6/06 A01H6/20 A01N59/16 A01N63/20 |
| X | NOURI MOHAMED ET AL: "Improving seed germination and seedling growth of Lepidium sativum with different priming methods under arsenic stress", ACTA ECOLOGICA SINICA, ELSEVIER, AMSTERDAM, NL, vol. 41, no. 1, 29 December 2020 (2020-12-29), pages 64-71, XP086496269, ISSN: 1872-2032, DOI: 10.1016/J.CHNAES.2020.12.005 [retrieved on 2020-12-29] * the whole document * | 1-4 | |
| A | CN 107 094 396 A (BENGBU JINNIUWAN AGRICULTURE TECH DEVELOPMENT CO LTD) 29 August 2017 (2017-08-29) | 3,4 | **TECHNICAL FIELDS SEARCHED (IPC)** A01H |
| A | US 2023/232835 A1 (HOLZFUSS CONSTANZE [DE] ET AL) 27 July 2023 (2023-07-27) | 3,4 | |

-/--

The present search report has been drawn up for all claims

| Place of search | Date of completion of the search | Examiner |
|---|---|---|
| Munich | 22 January 2025 | Kania, Thomas |

EPO FORM 1503 03.82 (P04C01)

Europäisches
Patentamt

European
Patent Office

Office européen
des brevets

# EUROPEAN SEARCH REPORT

Application Number

EP 24 02 0267

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/021204 A1 (KYOTO PREFECTURE [JP]; MEIJI SEIKA PHARMA CO LTD [JP]) 11 February 2016 (2016-02-11) ----- | 3,4 | |
| A | CN 110 352 657 A (INST OF INDUSTRIAL CROPS OF HEILONGJIANG ACADEMY OF AGRICULTURAL SCIEN) 22 October 2019 (2019-10-22) ----- | 1,2 | |
| A | JAFFAR NUR SULASTRI ET AL: "The potential of lactic acid bacteria in mediating the control of plant diseases and plant growth stimulation in crop production - A mini review", FRONTIERS IN PLANT SCIENCE, vol. 13, 13 January 2023 (2023-01-13), XP093241077, CH ISSN: 1664-462X, DOI: 10.3389/fpls.2022.1047945 ----- | 3,4 | |

**TECHNICAL FIELDS SEARCHED (IPC)**

The present search report has been drawn up for all claims

| Place of search | Date of completion of the search | Examiner |
|---|---|---|
| Munich | 22 January 2025 | Kania, Thomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .......................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 02 0267

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2025

| Patent document cited in search report | Publication date | Patent family member(s) | Publication date |
|---|---|---|---|
| CN 107094396 A | 29-08-2017 | NONE | |
| US 2023232835 A1 | 27-07-2023 | BR 112022023338 A2 | 27-12-2022 |
| | | CA 3181315 A1 | 23-12-2021 |
| | | CL 2022003524 A1 | 29-09-2023 |
| | | DE 202020103414 U1 | 16-09-2021 |
| | | EP 4164389 A1 | 19-04-2023 |
| | | US 2023232835 A1 | 27-07-2023 |
| | | WO 2021255033 A1 | 23-12-2021 |
| WO 2016021204 A1 | 11-02-2016 | EP 3178921 A1 | 14-06-2017 |
| | | JP WO2016021204 A1 | 25-05-2017 |
| | | US 2017231166 A1 | 17-08-2017 |
| | | WO 2016021204 A1 | 11-02-2016 |
| CN 110352657 A | 22-10-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARET W.** *Advances Nutrition*, 2013, vol. 4, 82-91
- **MARREIRO D**. *Antioxidants*, 2017, vol. 6, 24
- **PRASAD A.S.** *J. Am. Coll. Nutr.*, 2009, vol. 28, 257-265
- **GAMMOH N.Z.** *Nutrition and Immunity*, 2019, 127-158
- **PRASAD A.S.** *Mol. Med.*, 2008, vol. 14, 353-357
- **BLACK R.E.** *Lancet*, 2008, vol. 371, 243-260
- **READ S.A.** *Adv. Nutr.*, 2019, vol. 10, 696-710
- **WESSELS I**. *Front. Immunol.*, 2020, vol. 11, 1712
- **KUMSSA D.B.** *Sci. Rep.*, 2015, vol. 5, 1-11
- **LIM K.H.C.** *Nutrients*, 2013, vol. 5, 3184
- **CLEMENS S.** *Plant Sci.*, 2014, vol. 225, 52-57
- **BALMER A.** *Trends Plant Sci*, 2015, vol. 20, 7
- **HOSSAIN M.A.** *Front Plant Science*, 2015, vol. 6, 420
- **BAILLY C.** *Comptes Rendus Biologies*, 2008, vol. 331 (10), 806-814
- **KUBALA S.** *Post. Biol. Kom.*, 2013, vol. 40 (2), 215-230
- **DI GIROLAMO G.** ; **BARBANTI L.** *Italian J Agron*, 2012, vol. 7
- **IBRAHIM E.A.** *J Plant. Physiol.*, 2015, vol. 192, 38-46
- **SIVRITEPE H.O.** ; **SIVRITEPE N.** *Not. Bot. Hort. Agrobot.*, 2016, vol. 44 (2), 399-403
- **JISHA K.C.** *Acta Physiol. Plantarum*, 2012, vol. 35, 1981-1396
- **VENTURA L.** *Plant Physiol. Biochem.*, 2012, vol. 60, 196-206
- **WOJTYLA L.** *J Pant Physiol.*, 2016, vol. 203, 116-126
- **HOSSEIN M.A.** *Front Plant Sci.*, 2015, vol. 6, 420
- **SAVVIDES A.** *Trends Plant Sci.*, 2015, vol. 21 (4), 329-340
- **MAHDAVI B.** ; **RAHIMI A.** *EurAsia J BioSci*, 2013, vol. 7, 69-76
- **PAGNINI C.** *Microorganisms*, 2023, vol. 11 (6), 1381
- **GORBACH S.** *Nutrition Today*, 1996, vol. 31 (1), 2-4